# FASCICULE DE BREVET EUROPEEN

(11) **EP 0 695 537 B1**
(45) Date de publication et mention de la délivrance du brevet: **23.01.2002**
(21) Numéro de dépôt: 95440043.8
(22) Date de dépôt: 05.07.1995
(51) Int. Cl.: A61B 17/58, A61L 31/00

(54) **Dispositif de vis à tête filetée destinée à permettre la coaptation de deux fragments d'os**
Schraube mit Kopfgewinde welche eine Fixierung zweier Knochenfragmente erlaubt
Threaded head screw device for setting two bone fragments

(30) Priorité: 05.07.1994 FR 9408445
(43) Date de publication de la demande: 07.02.1996
(73) Titulaire: Depuy France, 69100 Villeurbanne (FR); Barouk, Louis Samuel, F-33370 Yvrac (FR)
(72) Inventeur: Barouk, Louis Samuel, 33370 Yvrac (FR); Augnagneur, Christian, 69007 Lyon (FR)
(74) Mandataire: Polus, Camille

(56) Documents cités:
- EP-A- 0 491 211
- WO-A-86/03666
- WO-A-91/09572
- WO-A-93/15682
- US-A- 5 019 079

## Description

La présente invention a pour objet, dans le domaine de la chirurgie orthopédique, un dispositif de vis à tête filetée destinée à permettre la coaptation de deux fragments d'os.

On connaît déjà, par le document US-A-4,175,555, une vis à plusieurs parties filetées, qui comporte en l'occurrence trois parties, à savoir une partie distale filetée, une longue partie intermédiaire lisse d'un diamètre compris entre celui des crêtes et celui de la racine de la partie distale, et une partie proximale filetée dont la racine a un diamètre sensiblement égal au diamètre des crêtes de la partie distale, le filetage de la partie proximale étant d'un pas plus petit que celui de la partie distale (voir également le document US-A-5 019 079).

Le vissage d'une telle vis dans un orifice préalablement foré et taraudé dans les deux fragments d'os à solidariser a pour effet, du fait de la différence des pas, une pénétration plus rapide de la partie distale, qui entraîne un resserrement des deux fragments d'os. Ce resserrement est accentué par les configurations des filets, qui comportent un bord perpendiculaire à l'axe de la vis, à savoir le bord inférieur pour la partie proximale, et le bord supérieur pour la partie distale .

La présente invention a plus particulièrement, mais non exclusivement, pour application le traitement de l'hallux valgus par l'ostéotomie dite Scarf, qui consiste généralement à sectionner l'os longitudinalement, puis à rapprocher les deux fragments d'os en les décalant latéralement et/ou longitudinalement.

Or la vis décrite dans le document sus-mentionné ne peut pas être utilisée dans cette technique chirurgicale pour plusieurs raisons . En effet, sa partie lisse doit se situer au niveau du trait de fracture, et pour cette raison elle est de préférence longue par rapport à l'ensemble de la vis.

Or, dans le cas du traitement de l'hallux valgus, les os sont petits, la vis doit donc l'être également, mais elle doit pouvoir s'ancrer au maximum, ce que ne permet pas la longue partie lisse.

De plus, également du fait de la petite taille des os traités, il est difficile de tarauder préalablement les orifices, mais la configuration des filets, notamment celui de la partie proximale, qui est inversé, ne permet pas qu'ils soient auto-taraudeurs.

La présente invention a pour objet un dispositif de vis à tête filetée permettant de remédier à ces divers inconvénients.

Un dispositif de vis selon l'invention se caractérise essentiellement d'une part en ce qu'il comporte une partie distale longue filetée sur pratiquement toute sa longueur, une partie proximale filetée courte et d'un diamètre supérieur à celui de ladite partie distale, le pas de cette dernière étant supérieur au pas de la partie proximale; d'autre part, en ce que les bords supérieurs des filets des deux parties filetées sont perpendiculaires à l'axe de la vis tandis que les bords inférieurs sont obliques; et d'autre part encore, en ce que les parties filetées comportent chacune à leur base au moins une entaille de taraudage.

Selon une caractéristique additionnelle du dispositif selon l'invention, un canal axial est pratiqué dans la vis pour permettre l'utilisation d'une broche de guidage.

Les avantages et les caractéristiques de la présente invention ressortiront plus clairement de la description qui suit et qui se rapporte au dessin annexé, lequel en représente un mode de réalisation non limitatif.

Dans le dessin annexé :
- la figure 1 représente une vue en élévation d'un dispositif de vis à tête filetée selon l'invention.
- la figure 2 représente une vue en coupe longitudinale selon un plan médian du même dispositif.
- la figure 3 représente une vue en plan de face de l'extrémité distale du même dispositif.

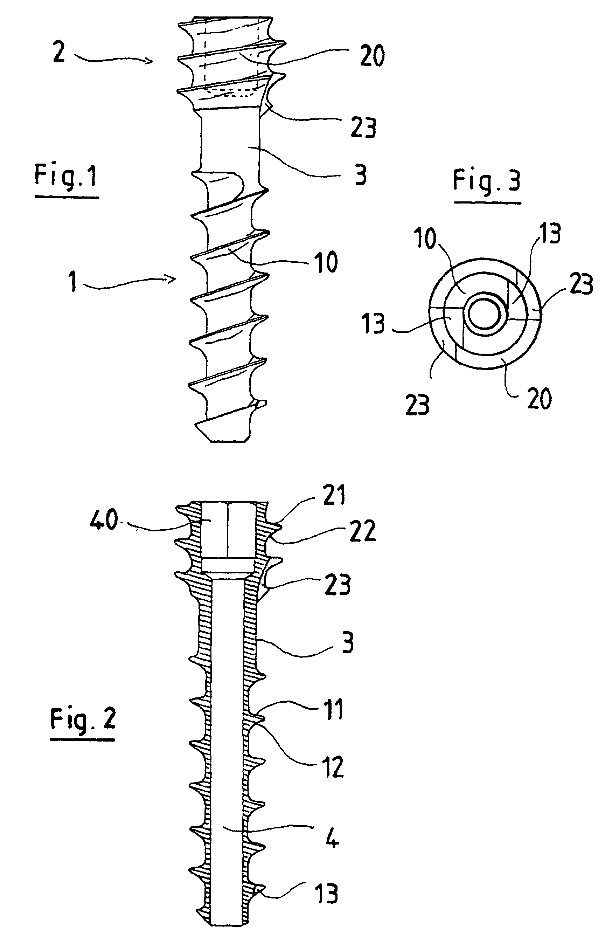

Si on se réfère à la figure 1, on peut voir qu'un dispositif de vis à tête filetée selon l'invention comporte une partie distale 1 longue et filetée, et une partie proximale 2 filetée courte et d'un diamètre supérieur à celui de la partie distale 1.

Les filetages sont de pas différents, le pas du filetage 10 de la partie distale 1 étant plus grand que le pas du filetage 20 de la partie proximale 2.

Si on se réfère également à la figure 2 on peut voir que les bords supérieurs 11 et 21 des filetages respectivement 10 et 20 sont perpendiculaires à l'axe de la vis, alors que les côtés inférieurs, respectivement 12 et 22, sont obliques.

Si on se réfère également à la figure 3, on peut voir que les extrémités inférieures des parties distale 1 et proximale 2 comportent chacune latéralement deux entailles, respectivement 13 et 23, diamétralement opposées, destinées à permettre un auto-taraudage, en association avec la configuration des filetages 10 et 20, à savoir l'obliquité des côtés inférieurs 12 et 22.

Sur les figures 1 et 2 on peut voir que la vis comporte, entre la partie distale 1 et la partie proximale 2, un tronçon 3 lisse, toutefois ce tronçon pourrait ne pas exister, ou être limité aux contraintes de fabrication.

Dans le traitement de l'hallux valgus par l'ostéotomie Scarf, du fait du décalage latéral d'un fragment d'os par rapport à l'autre, la vis est généralement enfoncée en périphérie des os, c'est-à-dire que la partie proximale et au moins l'extrémité de la partie distale sont ancrées dans la corticale, respectivement supérieure et inférieure, et que la partie centrale de la partie distale s'ancre costalement dans la corticale latérale.

Ainsi le filetage 10 de la partie distale 1 est pratiquement en contact sur toute sa longueur avec la corticale ce qui assure un bon ancrage, toutefois moins fort que celui réalisé par la partie proximale 2 et par l'extrémité de la partie distale 1, lequel permet un effet de resserrement.

Dans le traitement de l'hallux valgus il arrive également fréquemment, lorsqu'il s'agit de traiter le premier métatarsien, d'avoir, en plus des ancrages dans les corticales supérieure et inférieure, à traverser la corticale latérale, ce qui nécessite que la partie médiane de la vis soit filetée pour en assurer l'ancrage.

On peut également voir sur les figures 2 et 3 que la vis comporte un canal axial 4 prolongé par une empreinte femelle 40 de manoeuvrement, permettant le guidage de ladite vis au moyen d'un broche non représentée.

L'ensemble du dispositif selon l'invention peut être réalisé en n'importe quel matériau métallique biocompatible, par exemple du titane ou du TA 6 V, en alliage chrome-cobalt ou en acier inox, mais peut l'être aussi en un matériau biodégradable et/ou biocompatible, ou en un matériau minéral biointégrable, par exemple le phosphate de calcium ou l'hydroxyapatite.

La vis étant totalement enfouissable, elle ne nécessite pas d'être enlevée, en sorte qu'il est avantageux d'utiliser un matériau biointégrable.

Il va de soi que la présente invention ne saurait être limitée à la description qui précède d'un de ses modes de réalisation, susceptible de subir un certain nombre de modifications sans pour autant sortir du cadre de l'invention.

## Revendications

1. Dispositif de vis à tête filetée destinée à permettre la coaptation de deux fragments d'os, comportant une partie proximale (2) et une partie distale (1) filetées, la partie proximale (2) étant d'un diamètre supérieur au diamètre de la partie distale (1) et ayant un filetage d'un pas plus petit que celui de ladite-partie distale, les parties filetées (1,2) comportant chacune à leur base au moins une entaille (13, 23) de taraudage, les bords supérieurs (11) des filets du filetage distal étant perpendiculaires à l'axe de la vis, alors que leurs bords inférieurs sont obliques, **caractérisé** d'une part en ce que la partie distale (1) est filetée sur pratiquement toute sa longueur ; et d'autre part, en ce que les bords supérieurs (21) des filets de la partie filetée proximale (2) sont perpendiculaires à l'axe de la vis, tandis que leurs bords inférieurs (22) sont obliques.

2. Dispositif selon la revendication 1 **caractérisé en ce qu'**il comporte un canal axial (4) permettant l'utilisation d'une broche de guidage.

3. Dispositif selon la revendication 1 ou la revendication 2 **caractérisé en ce qu'**il est réalisé en un matériau biodégradable et/ou biocompatible.

4. Dispositif selon la revendication 1 ou la revendication 2 **caractérisé en ce qu'**il est réalisé en un matériau biointégrable.

## Patentansprüche

1. Schraube mit Kopfgewinde zum Befestigen zweier Knochenfragmente aneinander, mit einem gewindeten proximalen Abschnitt (2) und einem gewindeten distalen Abschnitt (1), wobei der proximale Abschnitt (2) einen größeren Durchmesser und eine kleinere Gewindesteigung als der distale Abschnitt (1) aufweist, wobei die gewindeten Abschnitte (1, 2) an ihrer Basis jeweils mindestens einen Einschnitt (13, 23) zum Gewindeschneiden aufweisen und wobei die oberen Flanken (11) der Gewindegänge des distalen Gewindes senkrecht zur Achse der Schraube verlaufen, während ihre unteren Flanken schräg sind, **gekennzeichnet** zum einen dadurch, dass der distale Abschnitt (1) im Wesentlichen über seine gesamte Länge gewindet ist und zum anderen dadurch, dass die oberen Flanken (21) der Gewindegänge des proximalen gewindeten Abschnitts (2) senkrecht zur Achse der Schraube verlaufen, während ihre unteren Flanken (22) schräg sind.

2. Vorrichtung nach Anspruch 1, **dadurch gekennzeichnet, dass** sie einen axialen Kanal (4) aufweist, der die Verwendung eines Führungsstifts ermöglicht.

3. Vorrichtung nach Anspruch 1 oder 2, **dadurch gekennzeichnet, dass** sie aus biologisch abbaubarem und/oder biologisch verträglichem Material hergestellt ist.

4. Vorrichtung nach Anspruch 1 oder 2, **dadurch gekennzeichnet, dass** sie aus biologisch integrierbarem Material hergestellt ist.

## Claims

1. Threaded head screw device intended to permit coaptation of two bone fragments, comprising a threaded proximal portion (2) and a threaded distal portion (1), the proximal portion (2) having a diameter greater than the diameter of the distal portion (1) and having a thread of a pitch which is smaller than that of said distal portion, the threaded portions (1, 2) each comprising at their base at least one internal thread recess (13, 23), the upper edges (11) of the threads of the distal threading being perpendicular to the axis of the screw, while their lower edges are oblique, **characterised** on the one hand in that the distal portion (1) is threaded over practically all of its length; and on the other hand, in that the upper edges (21) of the threads of the threaded proximal portion (2) are perpendicular to the axis of the screw, while their lower edges (22) are oblique.

2. Device according to claim 1, **characterised in that** it comprises an axial channel (4) which permits the use of a guide pin.

3. Device according to claim 1 or claim 2, **characterised in that** it is produced in a biodegradable and/or biocompatible material.

4. Device according to claim 1 or claim 2, **characterised in that** it is produced in a biointegrable material.
